# EUROPEAN PATENT SPECIFICATION

(11) **EP 1 605 772 B1**
(45) Date of publication and mention of the grant of the patent: **27.06.2007**
(21) Application number: 04721828.4
(22) Date of filing: 19.03.2004
(51) Int. Cl.: A23J 1/20, A23J 1/08, B01D 15/08

(54) **METHOD FOR HIGH THROUGHPUT VOLUMES IN THE FRACTIONATION OF BIO-MOLECULES BY CHROMATOGRAPHIC SYSTEMS**
VERFAHREN FÜR VOLUMEN MIT HOHEM DURCHSATZ BEI DER FRAKTIONIERUNG VON BIOMOLEKÜLEN DURCH CHROMATOGRAPHISCHE SYSTEME
PROCEDE POUR VOLUMES A HAUT RENDEMENT SERVANT AU FRACTIONNEMENT DE BIOMOLECULES PAR DES SYSTEMES CHROMATOGRAPHIQUES

(30) Priority: 21.03.2003 DK 200300443
(43) Date of publication of application: 21.12.2005
(73) Proprietor: UPFRONT CHROMATOGRAPHY A/S, DK-2100 Copenhagen Ö (DK)
(72) Inventor: HANSEN, Marie, Bendix, DK-2990 Niv (DK); LIHME, Allan, Otto, Fog, DK-3460 Birker d (DK)
(74) Representative: Plougmann & Vingtoft A/S
(86) International application number: PCT/DK2004/000187
(87) International publication number: WO 2004/082397

(56) References cited:
- WO-A-99/65586
- WO-A-02/096215
- GB-A- 1 363 783
- US-A- 5 719 053
- US-A- 5 986 063
- US-B1- 6 498 236
- ANSPACH F BIRGER ET AL: "Expanded-bed chromatography in primary protein purification" JOURNAL OF CHROMATOGRAPHY A, vol. 865, no. 1-2, 31 December 2000 (2000-12-31), pages 129-144, XP002285383 ISSN: 0021-9673

## Description

### FIELD OF INVENTION

The invention relates to an industrial scale chromatographic process for fractionation and isolation of bio-molecules from fluids, e.g. proteins from milk and whey in a cost-effective manner. The process allows for processing large volumes of fluid in a short time and for improved adsorbent efficiency by means of operating the process at high temperature and high flow rate.

### BACKGROUND OF THE INVENTION

Generally, a very broad range of different chromatographic processes for industrial scale fractionation and/or isolation of biological molecules, such as proteins, lipids, saccharides, lipo-proteins, poly-nucleotides, DNA, RNA, plasmids, virus, cells and cells constituents, are available.

When utilising chromatographic processes for industrial scale production, the production efficiency and economically consequences is a matter of strong considerations. Many attempts have been made in order to improve the efficiency of chromatographic processes, for instance by providing adsorbent particles of smaller sizes, increasing the surface of the adsorbent particle so as to improve the adsorptive capacity of the adsorbent towards a bio-molecule.

However, there is still a need for improving the efficiency of chromatographic processes for industrial scale production. In particular higher productivity may be needed upon isolating or fractionating bio-molecules from fluids with low content of the target bio-molecules. For example, the concentration of lactoferrin in bovine skimmed milk is usually low, typically between 80-200 mg/l depending on e.g. the pasteurisation process and other pre-treatment history of the skimmed milk.

Thus, a process allowing for higher productivity is of particular interest in fractionation and isolation of lactoferrin from milk or whey. WO 02/096215 relates to a method for fractionating lactoferrin from milk or whey using flow rates about 200 to 900 cm/hr. Furthermore, fractionation of immunoglobulins is of interest. WO 98/08603 relates to a method for isolation of immunoglobulins. Conventionally, these methodologies have been carried out using temperatures in the range of about 10°C.

In an industrial environment for production of bio-molecules, e.g. for production of food or food ingredients and bio-pharmaceuticals there is an Indispensable need for careful control of the microbiology in order to avoid contamination or breakdown of the target biomolecule product. In many instances the biomolecule target is further a delicate substance, e.g. an enzyme or other protein or peptide, a polynucleotide, a viral particle or other easily degradable substance that only have limited stability at elevated temperatures.
Therefore there is a general need to keep processing temperatures below 10-15 degrees Celsius since such low temperatures generally inhibit microbial growth while at the same time minimise the risk of deterioration of the target biomolecule.

Chromatographic adsorption processes are generally known to be able to accommodate a very broad range of processing temperatures and it is well-known in the art that Increased operating temperatures improve the mass transfer kinetics of a chromatographic system. Thus high operating temperatures are often applied in analytical HPLC columns for the analysis of low molecular weight compounds.

However, in an industrial environment with a need for control of microbial growth it is not optimal to operate the chromatographic adsorption process in a temperature interval in which common microorganisms grow the fastest. There has also been a prejudice in the field of chromatographic adsorption process against operating the chromatographic adsorption process at elevated temperatures since the target biomolecule at these high temperatures will have an increased risk of breakdown, oxidation, denaturation or other form of deterioration. An important drawback of the hitherto applied large-scale chromatographic adsorption processes in this context is that the operable flow rate through the column has been very low due to the physical constraints of typical packed bed adsorption columns in terms of increased back-pressure at elevated flow rates, compression and poor adsorption efficiency at high flow rates. The larger the scale of operation the more problematic the mentioned drawbacks will be.

Because of the physical constraints of a packed bed adsorption column an increase in operating temperature will not allow for significant increase of the operating flow rate without a very significant increase of the back-pressure over the column, which will be prohibitively costly to manage for many large-scale, commercial production applications.

The present investigators report herein a method for significant improvement of the productivity of chromatographic processes of industrial scale by providing means for operating the chromatographic processes with very high flow rates and still maintain highly efficient adsorption and integrity of the biomolecule under temperature conditions that inhibit microbial growth.. Thus, such processes may allow for more cost effective fractionation and/or isolation of bio-molecules of interest.

Patent 5,596,082 discloses an industrial process for isolation of lactoperoxidase and lactoferrin from milk and milk products with packed bed chromatography using a strong cation exchanger (SP Sepharose Big Beads from Amersham Biosclences). The chromatographic beads described for the process have a mean particle size in the range of 100- 300 microns and working flow rates in the range of 2000 - 3000 cm/hr may be used.

### SUMMARY OF INVENTION

The present invention relates to a chromatographic process capable of processing very large volumes of bio-molecule-containing fluids in a short time and capable of providing high productivity, while still achieving high purity and integrity of the biological molecule isolated by the process. This may be achieved by operating the chromatographic processes in a combination of high flow rates and high temperatures.

Thus, a primary aspect the invention relates to a general process for fractionating and/or isolation of one or more bio-molecule(s) from a bio-molecule-containing fluid, the process comprising the steps of:
a) optionally adjusting the pH of the bio-molecule-containing fluid;
b) bringing the bio-molecule-containing fluid to a temperature of at least 40°C;
c) applying a volume of said bio-molecule-containing fluid having a temperature of at least 40°C to a chromatographic column, such as an expanded bed adsorption column, comprising an adsorbent, at a linear flow rate of at least 1.500 cm/hour;
d) optionally washing the column;
e) eluting at least one bio-molecule from the adsorbent.

One object of the present invention is to provide an improved process for industrial-scale fractionation and/or isolation of proteins, such as lactoferrin, from suitable body fluids or fluids derived therefrom including milk and whey,

### DETAILED DESCRIPTION OF THE INVENTION

The present investigators provide herein evidence for that the combination of high operating temperature and high flow rate applied upon loading of the bio-molecule-containing fluids onto a chromatographic column and significantly improves the adsorptive capacity and the productivity of the adsorbent of the chromatographic column, while at the same time inhibit the microbial growth and keep the biomolecule intact. As can be derived from example 1, operating a chromatographic process at a temperature of 50°C instead of the conventional 10°C results in doubling of the adsorbent capacity of the adsorbent, i.e. the amount (g) of Lactoferrin adsorbed to 1 l of adsorbent was doubled. Furthermore, upon operating the chromatographic process at 50°C and with flow rates higher than conventional ones (from 1.500 cm/hr to 3.000 cm/hr), the volume of the bio-molecule-containing fluid that can be loaded onto the column increases significantly, while still achieving the same high adsorbent capacity (example 2). Thus, upon increasing the linear flow rate during loading of the blo-molecule-containing fluid onto the column, the productivity increases. Productivity might be regarded as the amount of blo-molecule that can be adsorbed to 1 litre of adsorbent in 1 hour. As can be seen from example 3, the process time is dramatically reduced upon operating the chromatographic process at higher temperatures, such as 50° in combination with higher linear flow rate, such as 2.100 cm/hr.

No breakdown or denaturation of the lactoferrin molecules could be detected by standard analytical procedures such as size-exclusion chromatography and sodiumdodecyl-gelelctrophoresis. The very high flow rate applied at the high temperature is believed to result in a low deterioration of the lactoferrin biomolecule due to the short time that passes from heating to the target temperature until the biomolecule is becoming adsorbed to the chromatographic adsorbent (a time span of a few seconds).

Since the chromatographic adsorption was performed as an expanded bed adsorption process there was no significant increase of backpressure over the adsorbent bed caused by the high flow-rate. This is in sharp contrast to what would be the case for a packed bed adsorption column with the same binding efficiency.

Thus, the combination of high temperatures and high flow rates seems to be a surprisingly promising approach in increasing the productivity of chromatographic systems, in particular systems of industrial scale where any reduction in costs may be of great commercial importance.

Accordingly, in a primary aspect the invention relates to a process for fractionating and/or isolation of one or more bio-molecule(s) from a bio-molecule-containing fluid comprising the steps of:
a) optionally adjusting the pH of the bio-molecule-containing fluid;
b) bringing the bio-molecule-containing fluid to a temperature of at least 40°C;
c) applying a volume of said bio-molecule-containing fluid having a temperature of at least 40°C to a chromatographic column, such as preferably an expanded bed column, comprising an adsorbent, said chromatographic column is operated with a linear flow rate of at least 1.500 cm/hour;
d) optionally washing the column;
e) eluting at least one bio-molecule from the adsorbent.

### Bio-molecules

As defined herein the term "bio-molecule" is intended to mean any molecule and entity that is obtainable from biological origin having a molecular weight of at least 1000 Daltons. As is to be understood, the bio-molecule may be obtained by use of synthetically means, gene technology and/or fermentation. Furthermore, the bio-molecule may be different to that of the biological origin because of derivatising of the bio-molecule. Thus, the term "bio-molecule is meant to encompass bio-molecules that are obtainable from biological origin and derivatives thereof. Typically, such bio-molecules are peptides, proteins, lipids, hormones, lipoproteins, polysaccharides, polynucleotides, bio-polymers or mixtures thereof. Furthermore, in some embodiments of the invention the term "bio-molecule" also encompasses entities obtainable from biological origin having a molecular weight of at least 20,000 D, e. g. DNA (plasmid DNA, chromosomal DNA, virus DNA), RNA such as virus RNA, or virus cells and constituents thereof, even bacteria cells and constituents thereof. The term "bio-molecule" is also meant to include cell constituents and cells.

It is contemplated that the process acquires practical importance for bio-molecules of higher molecular weight. Thus, in some embodiments of the invention, the one or more bio-molecule(s) has/have a molecular weight of at least 1500 Daltons, more preferably of at least 2000 Daltons.

As may be understood, the process of the invention may be applicable for a broad variety of bio-molecules as long as any adsorbent capable of binding the bio-molecule of interest is available. Therefore, in embodiments of the invention, the one or more blo-molecule(s) is/are selected from peptides, proteins, lipids, lipoproteins, polysaccharides, polynucleotides, plasmids, , DNA, RNA, viral particles, cell constituents, cells or combinations thereof.

In interesting embodiments thereof, the one or more bio-molecules is/are selected from:
o Proteins such as lactoferrin, immunoglobulins, β-lactoglobulin, α-lactalbumin, lactoperoxidase, patatin, protease inhibitors and other proteins from potatoes, enzymes, such as lysozym.
o Lipids such as phospholipids from milk.
o Polysaccharides, such as starches (maize and potato starch), and pectin's such as chitosans.
• polynucleotides
• plasmids
• DNA AND RNA
• VIRAL PARTICLES
• CELLS AND CELL CONSTITUENTS

### Bio-molecule-containing fluid

The process according to the present invention is targeted, at least in part, for industrial or large-scale fractionation processes where large volumes must be handled. Of interest are fluids containing bio-molecules in low content, such as fluids that otherwise may be discharged, e.g. process water containing Interesting blo-molecules, but in too low content in order to attract any commercial interest. However, bio-molecule-containing fluids that contain high amounts of bio-molecules are not anticipated by the present invention, and may constitute further interesting embodiments.

In the context of the present invention, the term "blo-molecule-containing fluid" is Intended to denote a fluid of biological origin or derived therefrom, which comprises at least one or more bio-molecule within the context of this invention to be fractionated, partially or wholly purified or isolated on an industrial or large scale. Typically, such fluids include body fluids or fluids derived therefrom including milk, skimmed milk, whey or other milk derived fluids; blood or fluids derived therefrom; plasma or fluids derived therefrom; serum or fluids derived therefrom; lymph or fluids derived therefrom; urine or fluids derived therefrom; egg white or fluids derived therefrom; or egg yolk or fluids derived therefrom. Also typically, the blo-molecule-containing fluid is denoted to include fermentation fluids; waste water; process water; plant extracts, such as fruit derived fluids; animal tissue extracts, such as fish derived fluids, animal blood plasma or animal serum; synthesis mixtures; and/or fluids derived therefrom.

Accordingly, in some embodiments of the invention the bio-molecule-containing fluid is selected from body fluids, fermentation fluids, wastewater, process water, plant extracts, animal tissue extracts, animal blood plasma, animal serum, synthesis mixtures and/or fluids derived therefrom.

In some embodiments of the invention, the bio-molecule-containing fluid is process water from the food and/or feed industry, e.g. process water from the production of starches, e.g. potato starch and/or maize starch. In still other embodiments, the bio-molecule-containing fluid is waste water comprising undesirable organic molecules, such as toxins, allergens, pesticides in that the waste water need to be purified from the containment of such undesirable organic molecules before being released to the environment or used for preparation of drinking water.

In presently Interesting embodiments, the bio-molecule-containing fluid is selected from the group comprising of milk, skimmed milk, whey or any other milk derived fluids.

### pH adjustment

As may be understood, the blo-molecule-containing fluid may before being loaded to a chromatographic column need an adjustment in pH depending on the protein of interest, the ligand chemistry, and the type of bio-molecule-containing fluid.

In some embodiments of the present invention the bio-molecule-containing fluid is pH adjusted prior to being applied to the adsorbent column in order to facilitate the capture of bio-molecule such as a protein by the adsorbent. This pH may be adjusted to a pH value selected in the entire pH range, preferably from pH 2-13, more preferably from pH 3-11.

### Chromatographic column

The chromatographic column to be used may be any kind suitable for either EBA (Expanded Bed Adsorption) or any non-packed bed adsorption system a combination thereof. The chromatographic column may be used in either a batch system or in a continuos system. Thus, in some embodiments of the invention, the chromatographic column is an expanded bed adsorption column and in still other embodiments, the chromatographic column is a stirred tank adsorption column.

In the present context the term " chromatographic column" relates to any kind of container, which can be supplied with at least one inlet and at least one outlet, for the application of the bio-molecule-containing fluid to the column and subsequent elution of one or more bio-molecule of interest.

The fact that the EBA technology generally can work efficiently with non-clarified fluids makes it attractive to implement for the isolation and fractionation of blo-mnlecules from fluids such as milk, whey fermentation fluids and process water. Compared to packed bed adsorption techniques EBA may offer a robust process comprising fewer steps resulting In increased yields and an improved process economy. Due to the expansion of the adsorbent bed during execution of an EBA process, EBA columns can be scaled up to industrial scale without any significant considerations to increased backpressure or breakdown of the process due to clogging of the system. This is often seen as a problem when using packed bed columns.

However, the present state of art within the EBA technology does not adequately address the solution of how to process high volumes of fluids, while still achieving high productivity.

General Expansion Bed Adsorption technology is known to the person skilled In the art and the process of the present invention may be adapted to the processes described in, for example, WO 92/00799, WO 92/18237, WO 97/17132, WO 98/33572, WO 98/08603, WO 00/57982, WO 01/58924, and WO 02/096215.

As may be understood, the process may be specific applicable to industrial scale systems. Thus, in interesting embodiments of the invention, the chromatographic column is a large-scale chromatographic column comprising at least 10 l of sedimented adsorbent, as may be determined as the amount (litre) of adsorbent settled when operating the column without flow. In still interesting embodiments thereof, the chromatographic column is a large-scale chromatographic column comprising from about 50 to 1000 l of sedimented adsorbent. Preferably, the amount of sedimented adsorbent is from about 100 to 1000 l, more preferably from about 200 to 900 l, most preferably from about 300 to 800 l.

Furthermore, for industrial scale production, the chromatographic column has a diameter of at least 10 cm, preferably of at least 20 cm, more preferably in the range of from about 50 cm to 200 cm, such as 100 to 150 cm.

### Temperature

As mentioned conventional methodologies within the field of fractionating and isolating bio-molecules often uses temperatures about 15 °C or lower, such as 10°C. However, one objection of the present invention is to utilise higher temperatures in chromatographic processes for isolation of bio-molecules, although high temperatures may under normal conditions negatively affect temperature sensitive bio-molecules. For instance enzymes may loose their enzymatic capacity upon being exposed to high temperatures, such as temperatures above 40°C.
In currently interesting embodiments of the invention, the chromatographic coiumn is operated at temperatures of at least 40°C, such as at least 45 °C, e.g. at least 50°C, such as at least 55 °C, e.g. at least 60°C, such as at least 65 °C, e.g. at least 70°C. However, an upper limit may exist, such as about 80°C when considering some bio-molecules that has tolerance for high temperatures, but may not resist temperatures above 80°C.. However, low molecular weight biomolecules, such as peptides, , may tolerate high temperatures, such as temperatures in the range of 40-100°C, such as in the range of 45-100°C, e.g. in the range of 45-90°C, such as in the range of 45-80°C, e.g. in the range of 45-70°C, such as in the range of 45-65°C, e.g. in the range of 50-100, such as in the range of 55-100°C, e.g. in the range of 60-100 °C, such as in the range of 65-100°C, e.g. in the range of 70-100°C. Therefore, in some embodiments the chromatographic column is operated at temperatures up to 100 °C.

Optionally the column and the adsorbent inside the column may be heated to the desired operating temperature before applying the biomotecule-containing fluid. Flushing the column with hot water or a buffer solution having the desired temperature may efficiently perform this temperature adjustment.

Optionally the column may be insulated or even heat-jacketed in order to maintain a constant temperature during the column operation. In many instances this may not be necessary, however, because the high flow of biomolecule containing fluid through the column is adequate to maintain the desired temperature.

In current interesting embodiments of the invention, the temperature of the bio-molecule-containing fluid is between 50 and 70 °C.

### Flow rate

One major advantage of the invention relates to the utility of high flow rates, rather than the conventional ones, which amount to about 200 cm/hr. According to the present invention, linear flow rates of from about 1.500 to 12.000 cm/hr may be applicable during loading of the bio-molecule-containing fluid to the chromatographic column. Preferably, the linear flow rate may be operated within the range from 1.800 to 10.000 cm/hr, such as within the range of 2.000 to 10.000 cm/hr, such as typically at linear flow rates of about 3000 to 7000 cm/hr.

The utilisation of higher flow rates allows for loading high volumes of bio-molecule-containing fluids within a shorter time than conventionally possible. However, this may depend on the size of column adapted. In current suitable embodiments of the invention, the volume to be applied onto the column is from about 2-3500 l/min.

In other terms, the efficiency of the process as defined herein may be expressed by the volume of bio-containing fluids that can be applied to 1 litre of adsorbent per hour. Thus, in some embodiments of the invention, the volume applied per litre of adsorbent in one hour is at least 50 I, preferably at least 100 I, and more preferably at least 150 l/min such as at least 200 l/min.

However, in packed bed methodology, high flow rates may results in high back pressures within the chromatographic column, thus affecting the performance of the chromatographic system, for example problems with leak and breakdown of equipment. The present investigators have found that the present process, which operates at high temperatures, such as above 45°C, allows for operating the chromatographic column with a pressure, as measured over the entire chromatographic column, of at most 10 bar. Typically the pressure is of at most 9, 8, 7, 6 or 5 bar, preferably of at most 4 bar, most preferably of at most 3 bar such as of at most 2.5 bar.

### Adsorbent

In the present context the term "adsorbent" relates to the entire bed present in the chromatographic column and the term "adsorbent particle" are used interchangeably with the term "particle" and relates to the individual single particles, which makes up the adsorbent.

Generally, the term "adsorbent" is meant to characterize any suitable adsorbent used in chromatographic processes such as adsorbents suitable for ion-exchange chromatography, protein A and Protein G affinity chromatography, other affinity chromatography, hydrophobic chromatography, reverse phase chromatography, thiophilic adsorption chromatography and mixed mode adsorption chromatography and the like.

The flow rate, the size of the particles and the density of the particles all have influence on the expansion of the fluid bed and it is important to control the degree of expansion in such a way to keep the particles inside the column. The degree of expansion may be determined as H/HO, where H0 is the height of the bed in packed bed mode (without flow) and H is the height of the bed in the expanded bed mode obtained when applying a flow of liquid to the column. In a preferred embodiment of the present invention the degree of expansion H/HO is in the range of 1.0-20, such as 1.0-10, e.g. 1.0-6, such as 1.2-5, e.g. 1.5-4 such as 4-6, such as 3-5, e.g. 3-4 such as 4-6. In an other preferred embodiment of the present invention the degree of expansion H/H0 is at most 1.0, such as at most 1.5, e.g. at most 2, such as at most 2.5, e.g. at most 3, such as at most 3.5, e.g. at most 4, such as at most 4.5, e.g. at most 5, such as at most 5.5, e.g. at most 6, such as at most 10, e.g. at most 20.

The particle size analysis performed and referred to throughout the description and the examples is based on an computerised image analysis of the bead population giving the number of particles at any given particle diameter in relation to the total number of particles analysed in the specific measurement. Typically the total number of particles analysed will be in the range of 250-500 particles. These particle size data may be transferred into the volume percent represented by each particle size by a routine mathematical transformation of the data, calculating the volume of each bead and relating this to the total volume occupied by all beads counted in the measurement.

The particle size distribution according to the invention is preferably defined so that more than 90% of the particles are present in a size ranging between 20% to 500% of the mean particle diameter. More preferable, 90% of the particles are present in a size ranging between 50-200% of the mean particle diameter, most preferable between 50-150% of the mean particle diameter.

Traditionally packed bed materials for isolation of biomolecules as defined herein have a mean diameter less than about 100 microns, which enables an efficient binding of the protein. Their disadvantage is their high flow resistance. It is not feasible to apply flow rates higher than 500 cm/hr, which is not a problem in analytical applications but for large scale processing it becomes a limiting factor.
At flow rates higher than 500 cm/hr the pressure drop over the column material will increase and the bed height will be the limiting factor. If large amounts of substances are to be processed the diameter of the column should be rather large. This requires construction of columns of high standard in order to meet the required adequate distribution of the substances and which resist the high-pressures. The cost of such column has a great impact on the process economy.

The level of clarification of the feed stream also affects the pressure drop. Traditional packed beds work as depth filters that can clog, resulting in increased pressure drop unless the feed is thoroughly clarified.

In the event where the chromatographic column is an EBA column, the density of the EBA adsorbent particle is found to be highly significant for the applicable flow rates in relation to the maximal degree of expansion of the adsorbent bed possible inside a typical EBA column (e.g. H/HO max 3-5) and must be at least 1.3 g/mL, more preferably at least 1.5 g/mL, still more preferably at least 1.8 g/mL, even more preferably at least 2.0 g/mL, most preferably at least 2.3 g/mL in order to enable a high productivity of the process and an acceptable degree of bed expansion.

As stated, the process of the invention may be operated with use of high flow rates, while still achieving high productivity and efficient adsorption of bio-molecules to the adsorbent. This may, at least in part, be due to the limitation in the mean particle diameter of the adsorbent particle. In a preferred embodiment of the present invention, the adsorbent particle has a mean particle size of at most 200 µm. Typically, the mean particle size is at most 150 µm, particularly at most 120 µm, more particularly at most 100 µm, even more particularly at most 90 µm, even more particularly at most 80 µm, even more particularly at most 70 µm. Typically the adsorbent particle has a mean particle size in the range of 40-150 µm, such as 40-120 µm, e.g. 40-100, such as 40-75, e.g. 40-50 µm.
By the term "mean particle size" is meant the particle size that 50% of the particles in the adsorbent has, as determined by the number of particles.

Alternatively expressed, in suitable embodiments of the invention the adsorbent is made of particles, wherein 50% of the number of particles has a particle size of at most 200 µm, particularly at most 175, 150, 120, 100, 90, 80 or at most 70 µm.

The particle size as referred to herein relates to the longest distance as can be measured on the particle.

In a combination of preferred embodiments, where the average particle diameter is 120 µm or less, the particle density is at least 1.6 g/mL, more preferably at least 1.9 g/mL. When the average particle diameter is less than 90 µm the density must be at least 1.8 g/mL or more preferable at least 2.0 g/mL. When the average particle diameter is less than 75 µm the density must be at least 2.0 g/mL, more preferable at least 2.3 g/mL and most preferable at least 2.5 g/mL.

In a preferred embodiment of the present Invention the adsorbent particle has a density of at least 1.5 g/ml, such as at least 1.8 g/ml, e.g. at least 2.0 g/ml, such as at least 2.5 g/ml, such as at least 2.6 g/ml, e.g. at least 3.0 g/ml, such as at least 3.5 g/ml, e.g. at least 4.0 g/ml, such as at least 5 g/ml, e.g. at least 7 g/ml, such as at least 10 g/ml, e.g. at least 15 g/ml.

The density of an adsorbent particle is meant to describe the density of the adsorbent in its fully solvated (e.g. hydrated) state as opposed to the density of a dried adsorbent.

The adsorbent particle used according to the invention must be at least partly permeable to the bio-molecular substance to be Isolated in order to ensure a significant binding capacity in contrast to impermeable particles that can only bind the target molecule on its surface resulting in relatively low binding capacity. The adsorbent particle may be of an array of different structures, compositions and shapes. The adsorbent particle may be constituted by for example a porous high density material such as porous ceramic beads, porous glass beads and porous zirconium oxide or a high density conglomerate as described below.

Thus, the adsorbent particles may be constituted of a number of chemically derivatised porous materials having the necessary density and binding capacity to operate at the given flow rates per se. In one embodiment the particles are either of the conglomerate type, as described in WO 92/00799, having at least two non-porous cores surrounded by a porous material, or of the pellicular type having a single non-porous core surrounded by a porous material.

In the present context the term "conglomerate type" relates to a particle of a particulate material, which comprises beads of core material of different types and sizes, held together by the polymeric base matrix, e.g. an core particle consisting of two or more high density particles held together by surrounding agarose (polymeric base matrix).

In the present context the term "pellicular type" relates to a composite of particles, wherein each particle consists of only one high density core material coated with a layer of the porous polymeric base matrix, e.g. a high density stainless steel bead coated with agarose.

Accordingly the term "at least one high density non-porous core" relates to either a pellicular core, comprising a single high density non-porous particle or it relates to a conglomerate core comprising more that one high-density non-porous particle.

The adsorbent particle, as stated, comprises a high-density non-porous core with a porous material surrounding the core, and said porous material optionally comprising a ligand at its outer surface.

In the present context the term "core" relates to the non-porous core particle or core particles present inside the adsorbent particle. The core particle or core particles may be incidental distributed within the porous material and is not limited to be located in the centre of the adsorbent particle.

The non-porous core constitutes typically of at most 50% of the total volume of the adsorbent particle, such as at most 40%, preferably at most 30%.

Examples of suitable non-porous core materials are inorganic compounds, metals, heavy metals, elementary non-metals, metal oxides, non metal oxides, metal salts and metal alloys, etc. as long as the density criteria above are fulfilled. Examples of such core materials are metal silicates metal borosilicates; ceramics including titanium diboride, titanium carbide, zirconium diboride, zirconium carbide, tungsten carbide, silicon carbide, aluminium nitride, silicon nitride, titanium nitride, yttrium oxide, silicon metal powder, and molybdenum disilide; metal oxides and sulfides, including magnesium, aluminium, titanium, vanadium, chromium, zirconium, hafnium, manganese, iron, cobalt, nickel, copper and silver oxide; non-metal oxides; metal salts, including barium sulfate; metallic elements, including tungsten, zirconium, titanium, hafnium, vanadium, chromium, manganese, iron, cobalt, nickel, indium, copper, silver, gold, palladium, platinum, ruthenium, osmium, rhodium and iridium, and alloys of metallic elements, such as alloys formed between said metallic elements, e.g. stainless steel; crystalline and amorphous forms of carbon, including graphite, carbon black and charcoal. Preferred non-porous core materials are tungsten carbamide, tungsten, steel and titanium beads such as stainless steel beads.

The porous material is a polymeric base matrix used as a means for covering and keeping multiple (or a single) core materials together and as a means for binding the adsorbing ligand.

The polymeric base matrix may be sought among certain types of natural or synthetic organic polymers, typically selected from i) natural and synthetic polysaccharides and other carbohydrate based polymers, including agar, alginate, carrageenan, guar gum, gum arabic, gum ghatti, gum tragacanth, karaya gum, locust bean gum, xanthan gum, agaroses, celluloses, pectins, mucins, dextrans, starches, heparins, chitosans, hydroxy starches, hydroxypropyl starches, carboxymethyl starches, hydroxyethyl celluloses, hydroxypropyl celluloses, and carboxymethyl celluloses; ii) synthetic organic polymers and monomers resulting in polymers, including acrylic polymers, polyamides, polyimides, polyesters, polyethers, polymeric vinyl compounds, polyalkenes, and substituted derivatives thereof, as well as copolymers comprising more than one such polymer functionally, and substituted derivatives thereof; and iii) mixture thereof.

A preferred group of polymeric base matrices are polysaccharides such as agarose.

From a productivity point of view it is important that the adsorbent is able to bind a high amount of the bio-molecule per volume of the adsorbent.

The preferred shape of a single adsorbent particle is substantially spherical. The overall shape of the particles is, however, normally not extremely critical, thus, the particles can have other types of rounded shapes, e.g. ellipsoid, droplet and bean forms. However, for certain applications (e.g. when the particles are used in a fluidised bed set-up), it is preferred that at least 95% of the particles are substantially spherical.

Preparation of the particulate material according to the invention may be performed by various methods known per se (e.g. by conventional processes known for the person skilled in the art, see e.g. EP 0 538 350 B1 or WO 97/17132. For example, by block polymerisation of monomers; suspension polymerisation of monomers; block or suspension gelation of gel-forming materials, e.g. by heating and cooling (e.g. of agarose) or by addition of gelation "catalysts" (e.g. adding a suitable metal ion to alginates or carrageenans); block or suspension cross-linking of suitable soluble materials (e.g. cross linking of dextrans, celluloses, or starches or gelatines, or other organic polymers with e.g. epichlorohydrin or divinyl sulphone); formation of silica polymers by acidification of silica solutions (e.g. block or suspension solutions); mixed procedures e.g. polymerisation and gelation; spraying procedures; and fluid bed coating of density controlling particles; cooling emulsions of density controlling particles suspended in polymeric base matrices in heated oil solvents; or by suspending density controlling particles and active substance in a suitable monomer or copolymer solution followed by polymerisation.

In a particularly suitable embodiment generally applicable for the preparation of the particulate material according to the invention, a particulate material comprising agarose as the polymeric base matrix and steel beads as the core material is obtained by heating a mixture of agarose in water (to about 95°C), adding the steel beads to the mixture and transferring the mixture to a hot oil (e.g. vegetable oils), emulsifying the mixture by vigorous stirring (optionally by adding a conventional emulsifier) and cooling the mixture. It will be appreciated by the person skilled in the art that the particle size (i.e. the amount of polymeric base matrix (here: agarose) which is incorporated in each particle can be adjusted by varying the speed of the mixer and the cooling process. Typically, following the primary production of a particle preparation the particle size distribution may be further defined by sieving and/or fluid bed elutriation.

The porous matrix, such as polymer agarose, is typically chemically derivatised with a low molecular weight compound referred to herein as the ligand and the adsorbent comprises a ligand with affinity to proteins. The ligand constitutes the adsorbing functionality of the adsorbent media or the polymeric backbone of the adsorbent particle has a binding functionality incorporated per se. Well-known ligand chemistries such as cation exchangers, e.g. sulphonic acid, have been proven to be efficient tools for purification of whey proteins such as lactoferrin and lactoperoxidase. These proteins are positively charged, even at neutral pH, and selective Interaction with a cation exchanger can be obtained. Other proteins require more sophisticated binding interaction with the ligand in order to obtain a selective adsorption.

Such affinity ligands, like the chargeable moieties, may be linked to the base matrix by methods known to the person skilled in the art, e.g. as described in "Immobilized Affinity Ligand Techniques" by Hermanson et al., Academic Press, Inc., San Diego, 1992. In cases where the polymeric base matrix do not have the properties to function as an active substance, the polymeric base matrix (or matrices where a mixture of polymers are used) may be derivatised to function as an active substances in the procedures of activation or derivatisation. Thus, materials comprising hydroxyl, amino, amide, carboxyl or thiol groups may be activated or derivatised using various activating chemicals, e.g. chemicals such as cyanogen bromide, divinyl sulfone, epichlorohydrin, bisepoxyranes, dibromopropanol, glutaric dialdehyde, carbodiimides, anhydrides, hydrazines, periodates, benzoquinones, triazines, tosylates, tresylates, and diazonium ions.

Specifically preferred methods for chemical derivatization and specific ligands applicable according to this invention is described in WO 98/08603.

In order to ensure an optimal adsorption strength and productivity of the adsorbent it has been found that the ligand concentration on the adsorbent is very significant. Thus, in a suitable embodiment, the adsorbent carries ligands for adsorption of the biomolecular substances in a concentration of at least 20 nM, such as at least 30 mM or at least 40 mM, preferably at least 50 mM and most preferably at least 60 mM.

A subset of adsorbents may be characterised in terms of their binding capacity to bovine serum albumin (BSA). This subset of adsorbents are typically those comprising a ligand selected from the group consisting of l) ligands comprising aromatic or heteroaromatic groups (radicals) of the following types as functional groups: benzoic acids such as 2-aminobenzoic acids, 3-aminobenzoic acids, 4-aminobenzoic acids, 2-mercaptobenzoic acids, 4-amino-2-chlorobenzoic acid, 2-amino-5-chlorobenzoic acid, 2-amino-4-chlorobenzoic acid, 4-aminosalicylic acids, 5-aminosalicylic acids, 3,4-diaminobenzoic acids, 3,5-diaminobenzoic acid, 5-aminolsophthalic acid, 4-aminophthalic acid; cinnamic acids such as hydroxy-cinnamic acids; nicotinic acids such as 2-mercaptonicotinic acids; naphthoic acids such as 2-hydroxy-1-naphthoic acid; quinolines such as 2-mercaptoquinoline; tetrazolacetic acids such as 5-mercapto-1-tetrazolacetic acid; thiadiazols such as 2-mercapto-5-methyl-1,3,4-thiadiazol; benzimidazols such as 2-aminobenzimidazol, 2-mercaptobenzimidazol, and 2-mercapto-5-nitrobenzimidazol; benzothiazols such as 2-aminobenzothiazol, 2-amino-6-nitrobenzothiazol, 2-mercaptobenzothiazol and 2-mercapto-6-ethoxybenzothiazol; benzoxazols such as 2-mercaptobenzoxazol; thiophenols such as thiophenol and 2-aminothiophenol; 2-(4-aminophenylthio)acetic acid; aromatic or heteroaromatic sulfonic acids and phosphonic acids, such as 1-amino-2-naphthol-4-sulfonic acid and phenols such as 2-amino-4-nitrophenol. It should be noted that the case where M is agarose, SP1 is derived from vinyl sulfone, and L is 4-aminobenzoic acid is specifically disclaimed in relation to the solid phase matrices according to the invention, cf. WO 92/16292, most preferably aminobenzoic acids like 2-amino-benzoic acid, 2-mercapto-benzoic acid, 3-aminobenzoic acid, 4-aminobenzoic acid, 4-amino-2-chlorobenzoic acid, 2-amino-5-chlorobenzoic acid, 2-amino-4-chlorobenzoic acid, 4-aminosalicylic acids, 5-aminosalicylic acids, 3,4-dlaminobenzolc acids, 3,5-diaminobenzoic acid, 5-5-aminolsophthalic acid, 4-aminophthalic acid; ii) ligands comprising 2-hydroxy-cinnamic acids, 3-hydroxy-cinnamic acid and 4-hydroxy-cinnamic acid iii) ligands comprising a carboxylic acid and an amino group as substituents such as 2-amino-nicotinic acid, 2-mercapto-nicotinic acid, 6-amino-nicotinic acid and 2-amino-4-hydroxypyrimidine-carboxylic acid iv) ligand comprising radicals derived from a benzene ring fused with a heteroaromatic ring system, e.g. a ligand selected from benzimidazoles such as 2-mercapto-benzimidazol and 2-mercapto-5-nitro-benzimidazol; benzothiazols such as 2-amino-6-nitrobenzothiazol, 2-mercaptobenzothiazol and 2-mercapto-6-ethoxybenzothiazol; benzoxazols such as 2-mercaptobenzoxazol;and v) ligands chosen from the group of thiophenols such as thiophenol and 2-aminothiophenol.

Within the embodiment wherein the ligand is selected from group i)-v), the adsorbents typically have a dynamic binding capacity of at least 10 g of biomolecular substance per litre, more preferably at least 20 g per litre, still more preferable at least 30 g per litre when tested according to the process conditions used in the relevant application. The binding capacity of the adsorbent may be determined in terms of its binding capacity to bovine serum albumin (BSA). The binding capacity is typically such that at least 10g/L of BSA binds according to test Method A.

Method A is a method used for determination of the bovine albumin binding capacity of selected adsorbents consisting of the following process:

Bovine serum albumin solution pH 4.0 (BSA pH 4.0): Purified bovine serum albumin (A 7906, Sigma, USA) is dissolved to a final concentration of 2 mg/ml in 20 mM sodium citrate pH 4.0. Adsorbents are washed with 50 volumes of 20 mM sodium citrate pH 4.0 and drained on a suction filter.

A sample of 1.0 ml suction drained adsorbent is placed in a 50 ml test tube followed by the addition of 30 ml of BSA, pH 4.0.

The test tube is then closed with a stopper and the suspension incubated on a roller mixer for 2 hours at room temperature (20-25 °C). The test tube is then centrifuged for 5 min. at 2000 RPM in order to sediment the adsorbent completely. The supernatant is then isolated from the adsorbent by pipetting into a separate test tube, avoiding the carry-over of any adsorbent particles and filtered through a small non-adsorbing 0.2 µm filtre (Millipore, USA). Following this a determination of the concentration of non-bound BSA in the supernatant is performed by measuring the optical density (OD) at 280 nm on a spectrophotometer.

The amount of BSA bound to the adsorbent is then calculated according to the following formula:
*mg BSA bound per ml suction drained adsorbent* =
(1-(OD of test supematant/OD of BSA starting solution)) x 60 mg BSA/ml adsorbent.

### Washing

In a preferred embodiment the washing liquid is water e.g. tap water, demineralised water, water produced by reverse osmosis or distilled water, aqueous buffers or other aqueous solutions with high ionic strength. In other preferred embodiments, the washing liquid is the sample collected at the column outlet during loading of the column with the bio-molecule-containing fluid; the so-called run through fraction. For example, if whey is loaded to the column, the washing liquid may be the collected "run-through sample" which essentially consist of the whey constituents that are not adsorbed to the column. In principle any bio-molecule-containing fluid that has been loaded to an expanded bed column and collected as the "run through fraction" can be applied as long as the "run though fraction" has the required ionic strength.

In a preferred embodiment of the present invention the flow rate used for the washing steps Involved is selected from the ranges outlined previously for conventional methodologies. These are generally much lower than the linear flow-rate used when loading the bio-molecule-containing fluid onto the column.

### Elution

The one or more bio-molecule(s) of interest is/are released from the adsorbent using an eluent such as a buffer or any other solution capable of changing for example the pH within the column and which produces a generally clear and concentrated solution of the one or more blo-molecule(s).

Appropriate eluents depend on the type of adsorbent and the elution may be performed by any method conventionally described and known in the art.

In some embodiments of the invention, wherein the bio-molecule is a protein, the elution of the adsorbed protein is performed with a solution, typically selected from the group consisting of dilute base, dilute acid, and water. In the embodiment wherein the eluting or washing step is performed with such a solution, the solution is dilute so as to minimise the amount of salt and other unwanted substances present in the eluted product.

Thus, in a preferred embodiment the dilute acid or base used for elution of the bio-molecule has a salt concentration of less than 50 mM, preferably less than 30 mM, even more preferable less than 20 mM. The determination of the salt concentration is performed directly on the eluate fraction containing the protein or proteins to be isolated without additional dilution of the eluate fraction. Common, low cost and non-toxic acids and bases are applicable. Specifically preferred are the bases sodium hydroxide (NaOH), potassium hydroxide (KOH), calcium hydroxide (Ca(OH)₂), ammonium hydroxide (NH₄OH).

In a preferred embodiment of the present invention the flow rate used for the elution step or steps involved is selected from the ranges outlined previously for applying the protein-containing mixture to the adsorbent column.

### EXAMPLES

### Example 1

Isolation of Lactoferrin (LF) from skimmed milk using expanded bed adsorption chromatography at 10°C versus 50°C:

Non-pasteurised skimmed milk with pH 6.6 was obtained from a local dairy company.

### Adsorbent

FastLine SP, product number 900-1600 UpFront Chromatography.
The adsorbent is based on agarose with tungsten carbide particles incorporated, density of approximately 2.9 g/ml, particle size in the range of 40-200 µm with a mean particle size of 80µm, strong cation exchanger comprising sulfonic acid groups.

### Pre-treatment of the non-pasteurised skimmed milk

For running the experiment at 10°C the skimmed milk was equilibrated to a temperature of 10°C and kept at 10°C during the experiment.

For running the experiment at 50°C the skimmed milk was pumped through a heat exchanger to reach 50°C before it was loaded onto the column. No pH adjustment was performed.

### Process parameters

The experiment was performed in a FastLine®300 expanded bed column (Ø=30 cm) product number 7300-0000, UpFront Chromatography.

The column was packed with a sedimented (packed) bed height (H₀) of 15 cm of adsorbent (10.6 l) and thoroughly equilibrated with demineralised water at 10°C and 50°C, respectively to create an expanded bed of the adsorbent having the desired temperature for the two experiments.

For both experiments 3180 l of the skimmed milk was loaded onto the column with a linear flow rate of 1.500 cm/hr.

For both experiments the column was washed with an aqueous buffer pH 6.5 containing 25 mM of sodium citrate and 0.15 M of sodium chloride. Following this wash lactoferrin was then eluted using a solution of 20mM sodium hydroxide, which was brought to pH 7 immediately after the elution by the addition of 1 M hydrochloric acid,

### Determination of Lactoferrin

The concentration of lactoferrin in the eluate was determined by Single Radial Immunodiffusion (RID) using goat anti-bovine lactoferrin from Bethyl Laboratories inc. (1 µl per cm²) as described in Scand. J. Immunol. Vol. 17, Suppl. 10, 41-56, 1983.
The concentration was calculated from a standard curve produced with well know concentrations of lactoferrin from Sigma (cat. no. L 9507).

### Results

The table below shows the volumes of skimmed milk and buffers loaded onto each column:

| Fraction | Process running at 10°C | Process running at 50°C |
|---|---|---|
| Volume of skimmed milk loaded, litres | 3180 | 3180 |
| Volume of washing solutions, litres | 174 | 210 |
| Elution of lactoferrin, litres | 105 | 114 |
| Total volume processed, litres | 3459 | 3504 |
| Process time, hr | 3.26 | 3.31 |

The actual flow rate through the columns is 100 l/hr/l of adsorbent.

The table below shows the results from the two experiments. (LF = Lactoferrin)

| Temperature °C | g LF in eluate | Adsorbent capacity g LF/I adsorbent | Expansion of adsorbent during load of skimmed milk, H/H₀ | Productivity g LF/I adsorbent/hr |
|---|---|---|---|---|
| 10 | 244 | 23 | 10 times | 7.1 |
| 50 | 461 | 44 | 4 times | 13.2 |

The results show that the productivity, as presented by the amount of Lactoferrin Isolated per litre adsorbent In one hour, is much higher when the process is operated at 50°C than when operated at 10°C.
No breakdown or denaturation of the lactoferrin molecules could be detected by standard analytical procedures such as size-exclusion chromatography and sodiumdodecyl-gelelctrophoresis (SDS_PAGE). Neither was there detected any significant growth of common bacteria in the run-through fractions and the lactoferrin eluate. The purity of the eluted lactoferrin was found to be higher than 95 % as determined by SDS-PAGE.

### Example 2

Isolation of lactoferrin from non-pasteurised skimmed milk using expanded bed chromatography at linear flow rates of 1,500, 2,100 or 3,000 cm/hr at 50°C.

All conditions except for the flow rates were the same as described in example 1.

### Results

The table below shows the volumes of skimmed milk and buffers loaded onto each column:

| Fraction | Process running at 1500 cm/hr | Process running at 2100 cm/hr | Process running at 3000 cm/hr |
|---|---|---|---|
| Volume of skimmed milk loaded, litres | 3180 | 3180 | 3180 |
| Volume of washing solutions, litres | 210 | 232 | 302 |
| Elution of lactoferrin, litres | 114 | 115 | 192 |
| Total volume processed, litres | 3504 | 3527 | 3674 |
| Process time, hr | 3.3 | 2.4 | 1.7 |

The table below shows the results from the three experiments. (LF = Lactoferrin)

| Flow rate cm/hr | Volume loaded l/hr/l adsorbent | g LF in eluate | Adsorbent capacity g LF/I adsorbent | Expansion of adsorbent during load of skimmed milk, H/H₀ | Productivity g LF/I adsorbent/hr |
|---|---|---|---|---|---|
| 1,500 | 100 | 466 | 44 | 3.9 times | 13.3 |
| 2,100 | 140 | 456 | 43 | 4.4 times | 17.9 |
| 3,000 | 200 | 445 | 42 | 8 times | 24.7 |

The results show that when operating the expanded bed column at 50°C and the linear flow rate increase from 1500 to 3000 cm/hr, the process productivity as well as the volume that can be loaded per hour per litre of adsorbent increases significantly. No breakdown or denaturation of the lactoferrin molecules could be detected by standard analytical procedures such as size-exclusion chromatography and SDS-PAGE. The purity of the eluted lactoferrin was found to be higher than 95 % as determined by SDS-PAGE and no significant microbial growth was observed during the experiment.

### Example 3

Isolation of lactoferrin from sweet whey using expanded bed adsorption chromatography at 16°C versus 50°C.

### Process parameters

The experiment was performed in a FastLine®300 expanded bed column (Ø=30 cm) product number 7300-0000, UpFront Chromatography.

The column was packed with 15 cm of adsorbent (10.6 l) and equilibrated with demineralised water at 16°C or 50°C respectively.

3180 1 of sweet whey adjusted by a heat exchanger to a temperature of 16°C or 50°C respectively was loaded onto the column with a linear flow rate of 900 and 1,500 cm/hr, respectively.

The column was washed with aqueous buffer pH 6.5 containing 25 mM of sodium citrate and 0.30 M of sodium chloride. Lactoferrin was then eluted using a solution of 20mM sodium hydroxide.

### Results

The table below shows the volume of sweet whey and buffers loaded onto each column:

| Fraction | Process at flow rate 900 cm/hr, 16°C | Process at flow rate 1500 cm/hr, 50°C |
|---|---|---|
| Volume of whey loaded, litres | 3180 | 3180 |
| Volume of washing solutions, litres | 75 | 180 |
| Elution of lactoferrin, litres | 73 | 150 |
| Total volume processed, litres | 3328 | 3510 |
| Process time, hr | 3.1 | 2.4 |

The table below shows the results from the two experiments. (LF = Lactoferrin)

| Flow rate cm/hr | T °C | g LF in eluate | Adsorbent capacity g LF/I adsorbent | Expansion of adsorbent, H/H₀ | Productivity g LF/I adsorbent/hr |
|---|---|---|---|---|---|
| 900 | 16 | 167 | 15.8 | 4 times | 5.1 |
| 1500 | 50 | 158 | 14.9 | 3.3 times | 6.2 |

The results indicate that it is possible to increase the flow rate from 1,500 to 2,100 cm/hr if the temperature is increased from 16 to 50°C and thereby obtain a higher productivity. No breakdown or denaturation of the lactoferrin molecules could be detected by standard analytical procedures such as size-exclusion chromatography and sodiumdodecyl-gelelctrophoresis

### Example 4

Isolation of whey proteins from sweet whey using expanded bed adsorption. at 1,500 cm/hr.
Sweet whey was obtained from a local dairy company, the pH was 6.3.

### Adsorbent

FastLine PRO, UpFront Chromatography.
The adsorbent is based on agarose with tungsten carbide particles incorporated, density of approximately 2.9 g/ml, particle size in the range of 40-200 µm with a mean particle size of 80µm. The adsorbent comprises a mixed mode ligand comprising an aromatic ring structure with a carboxylic acid substituent. The adsorbent binds molecules in the pH range of 3 to 6. The molecules are released by increasing the pH in the elution buffer to above 7.

### Pre-treatment of sweet whey

For running the experiment at 50 °C the sweet whey was pumped through a heat exchanger to reach 50 °C before it was loaded onto the column.
pH was adjusted to 4.7 with 1 M hydrochloric acid.

### Process parameters

The experiment was performed in a FastLine®300 expanded bed column (∅=30 cm) product number 7300-0000, UpFront Chromatography.
The column was packed with 15 cm of adsorbent (10.6 l) and equilibrated with demineralised water at 50°C.
160 l of sweet whey was loaded onto the column with a linear flow rate of 1,500 cm/hr. The volume flow through from the column was collected in three fractions.
Non-bound material was washed out with demineralised water (290 l). The bound proteins were eluted In two steps.
Step 1: 50 mM adipic acid, 0.1 mg/ml SDS (sodium dodecylsulfate) pH 5.3 (275 l).
Step 2: 20 mM NaOH (117 l).

### Results

Each fraction from the experiment was tested with SDS-PAGE to evaluate the content and nature of proteins.

### SDS PAGE

For SDS PAGE, Invitrogen SDS Page 4-20 % Tris-Glycine gel (cat no. EC6025) was used. Sample preparation: 25µl sample and 25µl sample buffer Tris-Glycine Invitrogen (cat no. LC2676) was mixed and boiled for 5 minutes in a water bath. The running buffer 0.024 M Tris (Sigma T1378), 0.19 M Glycine (Merck 5001901000), 0.1 % SDS (Sodium dodecyl sulphate, JT Baker 2811) pH 8.6 was added.

20 µl sample was applied in each analysis slot and the power was adjusted to give a current of 40 mA. When the blue line from the sample buffer reached one cm from the bottom of the gel the power was turned off and the gel was stained overnight in Invitrogens Colloidal Blue Staining Kit (cat. no. LC 6025) on a shaking table. The next day the gel was transferred into water and de-stained in water for 2 hours.

The SDS-PAGE shows that protein content is highly reduced in the three flow-through fractions from the column, the major part of the immunoglobulin G, bovine serum albumin, β-lactoglobulin and α-lactalbumin is bound to the adsorbent.
In elution step 1 (using 50 mM adipic acid, 0.1 mg/ml SDS (sodium dodecylsulfate) pH 5.3) all the bound β-lactoglobulin is recovered.
In elution step 2 using 20 mM NaOH all the bound immunoglobulin G, bovine serum albumin and α-lactalbumin is recovered. No breakdown or denaturation of the whey protein molecules could be detected by standard analytical procedures such as size-exclusion chromatography and sodiumdodecyl-gelelctrophoresis

### Example 5

Isolation of lactoferrin from non-pasteurised skimmed milk using expanded bed chromatography operated with high loading linear flow rates of 4800 and 6000 cm/hr and with temperature of 50°C.

All conditions except for the flow rates and the loading ratio were similar to those employed in Example 1

The non-pasteurised skimmed milk has an initial concentration of lactoferrin of 150 mg/l

### Results

The table shows the resulting yield of lactoferrin obtained from the process operated with high loading linear flow rates of 4800 or of 6000 cm/hr, respectively.

| Flow rate, cm/hr | Yield, mg LF/I skimmed milk | Load, l |
|---|---|---|
| 4800 | 135 | 4500 |
| 6000 | 120 | 6000 |

The table shows that it is possible to load the adsorbent utilising linear flow rates of 4800 cm/hr or 6000 cm/hr and still recover 90 % w/w and 80 % w/w, respectively, of the initial content of lactoferrin in skimmed milk.

### Example 6

Isolation of immunoglobulin G (IgG) from sweet whey utilising expanded bed adsorption chromatography operated with different column temperatures (40°C, 50°C, 55°C, 60°C and 65°C, respectlvely):

Sweet whey was obtained from cheese production.

### Adsorbent:

FastLine PRO, UpFront Chromatography.

### Pre-treatment of the non-pasteurised sweet whey:

For running the experiment at 40°C, 50°C, 55°C, 60°C and 65°C, respectively, the sweet whey was pumped through a heat exchanger to reach 40°C, 50°C, 55°C, 60°C and 65°C, respectively, before It was loaded onto the column. pH of the sweet whey was adjusted to 4.7 before being loaded onto the column.

### Process parameters

The experiment was performed in a FastLine®300 expanded bed column (0=30 cm) product number 7300-0000, UpFront Chromatography.

The column was packed with a sedimented bed height of 25cm of adsorbent (17.7 l) and then equilibrated with demineralised water to a temperature 40°C, 50°C, 55°C, 60°C and 65°C, respectively:

883 l of sweet whey was loaded through a heat exchanger onto the column with a linear flow rate of 1,800 cm/hr. The initial 5 times of column volumes of whey leaving the column was collected and used for the following wash of the adsorbent.

The adsorbent was washed with 5 times of the column volume collected during loading of the whey (88.5 l). Then the pH in the whey was adjusted to pH 5.3 before used for washing. Following the first wash of the column with the above-mentioned collected whey (Flow through fraction), the column was washed with demineralised water (89 l). Finally, IgG was eluted from the adsorbent using 20 mM of sodium hydroxide (61 l).

### Results

The concentration of IgG in the flow through fraction as well as in fluid collected during wash with demineralised water and elution with NaOH was determined by Single Radial Immunodiffusion (RID). Rabbit anti-bovine immunoglobuline from Dako Cytomation, Denmark (Cat. no.: Z247) was used (1 µl per cm2).

The amount of IgG recovered In the different fractions was determined as the percentage of IgG found in each fraction in relation to the total loaded amount of IgG, which was set to 100%.

All fractions from the experiments were tested with SDS-PAGE to evaluate the content and nature of the proteins.

The table below shows the results from the five experiments.

| Temperature °C | % IgG in the run through fraction | % IgG in the washed fraction | % IgG In the eluate |
|---|---|---|---|
| 40 | 25 | 5 | 65 |
| 50 | 25 | 5 | 65 |
| 55 | 15 | 0 | 75 |
| 60 | 15 | 0 | 75 |
| 65 | 25 | 5 | 40 |

The highest recovery of IgG in the eluate was achieved when the process was operated at temperatures of 55°C or at 60 °C.

The results from the SDS-PAGE shows that all eluates (from the experiments with column temperatures of 40°C, 50°C, 55°C, 60°C and 65°C, respectively) contain as the major proteins, IgG and Bovine serum albumine (BSA). As minor proteins, β-lactoglobulin and α-lactalbumin are seen.
Notably, it is observed that the eluates obtained from the experiments applying column temperatures higher than 55 °C contains a significant amount of α-lactalbumin.
The highest purity of IgG is obtained when running the column at 40° or 50°C.

### Example 7

Isolation of IgG from sweet whey utilising expanded bed adsorption chromatography operated at 50°C and utilising 2.5 or 5.0 times the column volume collected during loading of the whey onto the adsorbent (flow through volume) as the first washing liquid after loading the whey onto the adsorbent:

All test conditions, except for the volume of whey used for washing, bed height and loading volume of whey, were the same as described for Example 6:
Bed height is 50 cm
Loading ratio between whey and adsorbent is 1:40 (40 litres of whey per litre of adsorbent, amounts to 1414 litre)

### Results

The table below shows the results from the two experiments.

| Wash volume CV | % IgG in run through | % IgG I wash fraction | % IgG in eluate |
|---|---|---|---|
| 2.5 | 20 | 0 | 80 |
| 5 | 20 | 0 | 80 |

It is seen that the recovery of IgG is the same in both eluates, independently of the volume of the first washing liquid.

The results from SDS-PAGE shows that different proteins is present in eluates resulting from the experiments utilising 2.5 times and 5 times, respectively, of the column volume of sweet whey for the first wash of the adsorbent. For example, β-lactoglobulin is present in the eluate resulting from the "2.5" experiment, whereas this protein is not detected when utilising 5 times the column volume of sweet whey as the washing liquid for the first wash of the adsorbent.

### Example 8

Isolation of IgG from sweet whey utilising expanded bed adsorption chromatography operated with column temperatures of 50°C and two different bed heights, 25 cm and 50 cm respectively:

All conditions except for the bed height were the same as described in Example 6:

### Results

The table below shows the results from the two experiments.

| Bed height (cm) | % IgG in the run through collected sample |
|---|---|
| 25 | 35 |
| 50 | 25 |

An increase of the bed height from 25 to 50 cm decreases the amount of IgG in the flow through sample collected during loading of the whey onto the adsorbent with 10 %, which leads to a higher yield from the raw material.

### Example 9

Isolation of IgG from sweet whey utilising expanded bed adsorption chromatography at 50°C. Different volumes of sweet whey was loaded onto the adsorbent; load ratio 1:30, 1:40 and 1:50 respectively:

All conditions, except with respect to bed height (50 cm) and the loading ratio, were the same as described for Example 6:

### Results

The table below shows the results from the three experiments.

| Loading ratio volume of whey (I)/ volume of adsorbent (I) | Recovery of IgG (%) in the collected "run through fraction" |
|---|---|
| 30 | 15 |
| 40 | 15 |
| 50 | 30 |

The amount of IgG recovered in the fraction collected as the flow through fraction upon loading the whey onto the adsorbent increases with the increasing loading ratios.

### Example 10

Isolation of IgG from sweet whey utilising expanded bed adsorption chromatography at 50°C. The sweet whey was loaded onto the adsorbent using different linear flow rates: 1200 cm/hr, 1800 cm/hr and 2400 cm/hr, respectively:

All conditions, except for the flow rate, were the same as described in example 6:

### Results

The table below shows the results from the three experiments.

| Flow rate during loading (cm/hr) | Recovery of IgG (%) in the collected "run through fraction" |
|---|---|
| 1200 | 7.5 |
| 1800 | 35 |
| 2400 | 50 |

The amount of IgG in the "run through fraction" increases with increasing flow rates.

### Example 11

Polish of bovine IgG, isolated from sweet whey, In order to enhance the purity. The IgG-containing eluate (product) obtained from the FastLine PRO adsorbent is passed through a weak anion exchanger in order to remove bovine serum albumine. The weak anion exchanger binds the bovine serum albumin for which reason the remaining IgG is collected in the flow through fraction. This result in a sample highly purified with respect to IgG.

The IgG-containing eluate from Example 7 (washed with 5 times the column volume of the collected "flow through fraction" of whey) was used.

The process was performed with an expanded bed system at room temperature at a linear flow rate at 900 cm/hr.

### Adsorbent

FastLine PEI, UpFront Chromatography.

### Pre-treatment of the IgG product

The pH of the IgG product was adjusted to pH 7.0 with 1 M hydrochloric acid before being loaded onto the column.

### Process parameters

The experiment was performed in a FastLine®100 expanded bed column (Ø=10 cm) product number 7100-0000, UpFront Chromatography.

The column was packed with a sedimented bed height of 50 cm of adsorbent (3.9 l) and equilibrated with 1 M NaOH and demineralised water.

121 l of IgG product containing BSA was loaded onto the column with a linear flow rate of 900 cm/hr.

### Results

The flow through fraction was collected and tested for content of BSA and IgG

### Determination of BSA and IgG

The concentration of BSA and IgG in the flow through fraction (IgG product) was determined by Single Radial Immunodiffusion (RID).
Rabbit anti-bovine serum albumin from Dako Cytomation (Cat. no.: Z229) was used (0.75 µl per cm2).
Rabbit anti-bovine immunoglobulin from Dako Cytomation (Cat. no.: Z247) was used (1 µl per cm2).
The amount of BSA and IgG in the flow through is defined as a percentage of BSA and IgG compared with the total load of BSA and IgG that equals a 100 %.

All fractions from the experiments were tested with SDS-PAGE to evaluate the content and nature of the proteins.

The SDS-PAGE shows that 90 % of the BSA was removed from the IgG product using the above-mentioned weak anion exchanger. The purified IgG-fraction is estimated to have a purity higher than 80 % with respect to IgG.

### Example 12

Isolation of lysozyme from egg white utilising expanded bed adsorption chromatography at 50°C. The egg white was loaded onto the adsorbent at a flow rate of 1500 cm/hr.

Egg white was obtained from a local egg industry.

### Adsorbent

FastLine SP, UpFront Chromatography.

### Pre-treatment of the egg white:

For running the experiment at 50°C the egg white was pumped through a heat exchanger to reach 50°C before it was loaded onto the column.

The pH of the egg white was adjusted to pH 7 with 1 M hydrochloric acid before being loaded onto the column.

### Process parameters:

The experiment was performed in a FastLine®300 expanded bed column (Ø=30 cm) product number 7300-0000, UpFront Chromatography.

The column was packed with a sedimented bed height of 25cm of adsorbent (17.7 l) and equilibrated with demineralised water, at 50°C.

142 l of egg white was loaded onto the column with a linear flow rate of 1,500 cm/hr.

The adsorbent was first washed with 50 mM of sodium chloride (177 l). Then, the lysozyme was eluted from the adsorbent using 20 mM sodium hydroxide (195 l).

### Results

The activity of the lysozyme was determined using Micrococcus lysodeikticus cells, Sigma Chemicals, USA (Shugar, David. Biochimica et Biophysica Acta 1952, 8,302-309)

The eluate contained 525 g of highly purified lysozyme. The yield of lysozyme was high approximately 100 %.
The purity of the lysozyme was shown to be high, estimated to be higher than 90 % as demonstrated using SDS-PAGE.

### Example 13

Isolation of potato proteins from potato juice utilising expanded bed adsorption chromatography operated at 50°C. The potato juice was loaded onto the adsorbent at a flow rate of 1500 cm/hr.

Potato juice was produced from washed potatoes. The potatoes were blended for 5 minutes, 30 ml of sodium sulphite was added per 2 kg of potatoes to prevent enzymatic browning of the juice. The blended potatoes were pressed through 100µm nylon net and the juice was collected.

### Adsorbent

FastLine PRO, UpFront Chromatography.

### Pre-treatment of the potato juice

For running the experiment at 50°C, the potato juice was pumped through a heat exchanger to reach 50°C before It was loaded onto the column.

The pH of the juice was adjusted to pH 4.5 with 1 M hydrochloric acid before being loaded onto the column.

### Process parameters:

The experiment was performed in a FastLine®300 expanded bed column (∅=30 cm) product number 7300-0000, UpFront Chromatography.

The column was packed with a sedimented bed height of 65cm of adsorbent (46 1) and equilibrated with demineralised water, at 50°C. 610 l of potato juice was loaded onto the column with a linear flow rate of 1,500 cm/hr.

The adsorbent was washed with 10 mM sodium citrate pH 4.5 (322 l). The proteins were eluted from the adsorbent with 10 mM sodium hydroxide (230 l).

### Results:

The eluate was freeze-dried and the protein content was determined by Kjeldahl.

After freeze drying 525 g of potato powder was recovered. Protein analysis showed that the protein content was 95 %.

## Claims

1. A process for isolation of one or more bio-molecule(s) from a bio-molecule-containing fluid comprising the steps of:
a) optionally adjusting the pH of the bio-molecule-containing fluid;
b) bringing the bio-molecule-containing fluid to a temperature of at least 40°C;
c) applying a volume of said bio-molecule-containing fluid having a temperature of at least 40°C to an expanded bed adsorption column comprising an adsorbent, said expanded bed column is operated with a linear flow rate of at least 1.500 cm/hour;
d) optionally washing the column;
e) eluting at least one bio-molecule from the adsorbent.

2. The process according to claim 1, wherein the expanded bed column is a large-scale column comprising at least 10 1 of sedimented adsorbent.

3. The process according to any one of claims 1 or 2, wherein the expanded bed column is a large-scale column comprising from about 50 to 1000 l of sedimented adsorbent, preferably from about 100 to 1000 l of adsorbent, more preferably from about 200 to 900 l of adsorbent, most preferably from about 300 to 800 l of adsorbent.

4. The process according to any of the preceding claims, wherein the expanded bed column has a diameter of at least 10 cm, preferably of at least 20 cm, more preferably in the range of from about 50 cm to 200 cm, such as 100 to 150 cm.

5. The process according to any of the preceding claims, wherein the one or more bio-molecule(s) has a molecular weight of at least 1000 Daltons, preferably of at least 1500 Daltons, more preferably of at least 2000 Daltons.

6. The process according to any of the preceding claims, wherein the one or more bio-molecule(s) is/are selected from the group consisting of peptides, proteins, lipids, lipoproteins, polysaccharides, DNA, RNA, plasmids, , polynucleotides, viral particles, cell constituents, cells and combinations thereof.

7. The process according to claim 6, wherein said proteins is selected from the group consisting of lactoferrin, β-lactoglobulin, α-lactalbumin, immunoglobulins and lactoperoxidase.

8. The process according to any of the preceding claims, wherein the bio-molecule-containing fluid is selected from the group consisting of body fluids, fermentation fluids, waste water, process water, plant extracts, animal tissue extracts, animal blood plasma, animal serum, synthesis mixtures and fluids derived therefrom.

9. The process according to claim 8, wherein the body fluid is selected from the group consisting of milk, plasma, urine, egg white and fluids derived therefrom.

10. The process according to any of the preceding claims, wherein the adsorbent consists of adsorbent particles wherein 50% of the number of particles has a particle size of at most 200 µm, preferably at most 175, 150, 120, 100 or 80µm.

11. The process according to any of the preceding claims, wherein the adsorbent consists of adsorbent particles wherein 50% of the number of particles has a particle size of at most 200 µm, such as at most 150 µm, particularly at most 120 µm, more particularly at most 100 µm, even more particularly at most 90 µm, even more particularly at most 80 µm, even more particularly at most 70 µm.

12. The process according to any one of preceding claims, wherein the adsorbent particle has a density of at least 1.5 g/ml.

13. The process according to any of the preceding claims, wherein the linear flow-rate is from about 1.500 to 12.000 cm/hr, preferably from about 1.800 to 10.000 cm/hr, such as about 3000 cm/hr.

14. The process according to any of the preceding claims, wherein the volume applied is from about 2-3500 I/min.

15. The process according to any of the preceding claims, wherein the volume applied per litre of adsorbent In one hour is at least 50 I, preferably at least 100 I, more preferably at least 150 I/min.

## Patentansprüche

1. Verfahren zur Isolierung von einem oder mehreren Bio-Molekül(en) aus einem bio-molekülhaltigen Fluid, wobei das Verfahren folgende Schritte umfasst:
a) wahlweise Einstellung des pH-Wertes des bio-molekülhaltigen Fluids;
b) Veranlassung der Temperatur des bio-molekülhaltigen Fluids auf mindestens 40°C zu steigen;
c) Anbringung einer Menge des bio-molekülhaltigen Fluids mit einer Temperatur von mindestens 40°C auf eine expanded Bett-Adsorptionssäule, die ein Adsorbens umfasst, wobei die expanded Bettsäule mit einer linearen Fließgeschwindigkeit von mindestens 1500 cm/Stunde betrieben wird;
d) wahlweises Waschen der Säule;
e) Eluierung von mindestens einem Bio-Molekül vom Adsorbens.

2. Verfahren nach Anspruch 1, worin die expanded Bettsäule eine großformatige Säule ist, die mindestens 10 1 sedimentiertes Adsorbens umfasst.

3. Verfahren nach irgendeinem der Ansprüche 1 oder 2, worin die expanded Bettsäule eine großformatige Säule ist, die ca. 50 bis 1000 1 sedimentiertes Adsorbens, vorzugsweise ca. 100 bis 1000 l Adsorbens, noch bevorzugter ca. 200 bis 900 l Adsorbens, am meisten bevorzugt ca. 300 bis 800 l Adsorbens umfasst.

4. Verfahren nach irgendeinem der vorhergehenden Ansprüche, worin die expanded Bettsäule einen Durchmesser von mindestens 10 cm, vorzugsweise mindestens 20 cm, noch bevorzugter im Bereich von ca. 50 cm bis 200 cm, wie z.B. 100 bis 150 cm, hat.

5. Verfahren nach irgendeinem der vorhergehenden Ansprüche, worin das eine oder mehrere Bio-Molekül(e) ein Molekulargewicht von mindestens 1000 Daltons, vorzugsweise mindestens 1500 Daltons, noch bevorzugter mindestens 2000 Daltons hat/haben.

6. Verfahren nach irgendeinem der vorhergehenden Ansprüche, worin das eine oder mehrere Bio-Molekül(e) aus der Gruppe bestehend aus Peptiden, Proteinen, Lipiden, Lipoproteinen, Polysacchariden, DNA, RNA, Plasmiden, Polynukleotiden, Viruspartikeln, Zellenbestandteilen, Zellen und Kombinationen davon gewählt ist/sind.

7. Verfahren nach Anspruch 6, worin die Proteine aus der Gruppe bestehend aus Lactoferrin, β-Lactoglobulin, α-Lactalbumin, Immunoglobulinen und Lactoperoxidase gewählt sind.

8. Verfahren nach irgendeinem der vorhergehenden Ansprüche, worin das bio-molekülhaltige Fluid aus der Gruppe bestehend aus Körperflüssigkeiten, Fermentierungsflüssigkeiten, Abwasser, Prozesswasser, Pflanzenextrakten, Gewebeextrakten aus Tieren, Blutplasma aus Tieren, Serum aus Tieren, Synthesemischungen und daraus abgeleiteten Flüssigkeiten gewählt ist.

9. Verfahren nach Anspruch 8, worin die Körperflüssigkeit aus der Gruppe bestehend aus Milch, Plasma, Harn, Eiweiß und daraus abgeleiteten Flüssigkeiten gewählt ist.

10. Verfahren nach irgendeinem der vorhergehenden Ansprüche, worin das Adsorbens aus Adsorptionspartikeln besteht, worin 50% der Anzahl von Partikeln eine Partikelgröße von höchstens 200 µm, vorzugsweise höchstens 175, 150, 120, 100 oder 80µm hat.

11. Verfahren nach irgendeinem der vorhergehenden Ansprüche, worin das Adsorbens aus Adsorptionspartikeln besteht, worin 50% der Anzahl von Partikeln eine Partikelgröße von höchstens 200 µm, wie z.B. höchstens 150 µm, insbesondere höchstens 120 µm, insbesondere höchstens 100 µm, insbesondere höchstens 90 µm, insbesondere höchstens 80 µm, insbesondere höchstens 70 µm hat.

12. Verfahren nach irgendeinem der vorhergehenden Ansprüche, worin das Adsorptionspartikel eine Dichte von mindestens 1,5 g/ml hat.

13. Verfahren nach irgendeinem der vorhergehenden Ansprüche, worin die lineare Fließgeschwindigkeit von ca. 1,500 bis 12,000 cm/Stunde, vorzugsweise von ca. 1,800 bis 10,000 cm/Stunde, wie z.B. ca. 3000 cm/Stunde ist.

14. Verfahren nach irgendeinem der vorhergehenden Ansprüche, worin die angebrachte Menge von ca. 2-3500 l/min ist.

15. Verfahren nach irgendeinem der vorhergehenden Ansprüche, worin die angebrachte Menge pro Liter Adsorbens in einer Stunde mindestens 50 I, vorzugsweise mindestens 100 1, noch bevorzugter mindestens 150 1/min ist.

## Revendications

1. Procédé de récolte d'une ou plusieurs biomolécule(s) d'un fluide contenant des biomolécules, comprenant les étapes de:
a) ajuster éventuellement le pH du fluide contenant des biomolécules;
b) porter le fluide contenant les biomolécules à une température d'au moins 40°C;
c) appliquer un volume du fluide contenant les biomolécules ayant une température d'au moins 40°C à une colonne d'adsorption à lit expansé comprenant un adsorbent, ladite colonne à lit expansé étant opérée à une vitesse linéaire d'au moins 1500 cm/h;
d) laver éventuellement la colonne;
e) éluer au moins une biomolécule de l'adsorbent.

2. Procédé selon la revendication 1, dans lequel la colonne à lit expansé est une colonne à grande échelle comprenant au moins 10 l d'adsorbent sédimenté.

3. Procédé selon l'une quelconque des revendications 1 ou 2, dans lequel la colonne à lit expansé est une colonne à grande échelle comprenant d'environ 50 à 1000 l d'adsorbent sédimenté, de préférence d'environ 100 à 1000 l d'adsorbent, encore plus préférablement d'environ 200 à 900 l d'adsorbent, le plus préférablement d'environ 300 à 800 l d'adsorbent.

4. Procédé selon l'une quelconque des revendications précédentes, dans lequel la colonne à lit expansé a un diamètre d'au moins 10 cm, de préférence d'au moins 20 cm, plus préférablement dans la plage d'environ 50 cm à 200 cm, tel que 100 à 150 cm.

5. Procédé selon l'une quelconque des revendications précédentes, dans lequel la une ou plusieurs biomolécule(s) a un poids moléculaire d'au moins 1000 Daltons, de préférence d'au moins 1500 Daltons, plus préférablement d'au moins 2000 Daltons.

6. Procédé selon l'une quelconque des revendications précédentes, dans lequel la une ou plusieurs biomolécule(s) est/sont choisie(s) dans le groupe constitué de peptides, protéines, lipides, lipoprotéines, polysaccharides, ADN, ARN, plasmides, polynucléotides, particules virales, constituants cellulaires, cellules et leurs mélanges.

7. Procédé selon la revendication 6, dans lequel les protéines sont choisies dans le groupe constitué de la lactoferrine, la β-lactoglobuline, l'α-lactalbumine, les immunoglobulines et la lactoperoxidase.

8. Procédé selon l'une quelconque des revendications précédentes, dans lequel le fluide contenant les biomolécules est choisi dans le groupe constitué des fluides corporels, les fluides de fermentation, les eaux résiduaires, les eaux de traitement, les extraits de plantes, les extraits de tissus animal, le plasma sanguin animal, le sérum animal, les mélanges de synthèse et leurs fluides dérivés.

9. Procédé selon la revendication 8, dans lequel le fluide corporel est choisi dans le groupe constitué du lait, le plasma, l'urine, l'ovalbumine et leurs fluides dérivés.

10. Procédé selon l'une quelconque des revendications précédentes, dans lequel l'adsorbent est constitué de particules d'adsorbent, dont 50% du nombre de particules a une dimension de particule d'au plus 200 µm, de préférence au plus 175, 150, 120, 100 ou 80 µm.

11. Procédé selon l'une quelconque des revendications précédentes, dans lequel l'adsorbent est constitué de particules d'adsorbent, dont 50% du nombre de particules a une dimension de particule d'au plus 200 µm, telle qu'au plus 150 µm, en particulier au plus 120 µm, plus particulièrement au plus 100 µm, encore plus particulièrement au plus 90 µm, et encore plus particulièrement au plus 80 µm, encore plus particulièrement au plus 70 µm.

12. Procédé selon l'une quelconque des revendications précédentes, dans lequel la particule adsorbante a une densité d'au moins 1,5 g/ml.

13. Procédé selon l'une quelconque des revendications précédentes, dans lequel la vitesse linéaire est d'environ 1500 à 12.000 cm/h, de préférence d'environ 1800 à 10.000 cm/h, telle qu'environ 3000 cm/h.

14. Procédé selon l'une quelconque des revendications précédentes, dans lequel le volume appliqué est d'environ 2-3500 l/min.

15. Procédé selon l'une quelconque des revendications précédentes, dans lequel le volume appliqué par litre d'adsorbent en une heure est d'au moins 50 I, de préférence au moins 100 I, plus préférablement au moins 150 l/min.
